# EUROPEAN PATENT SPECIFICATION

(11) **EP 4 589 292 B1**
(45) Date of publication and mention of the grant of the patent: **04.11.2026**
(21) Application number: 25150108.6
(22) Date of filing: 02.01.2025
(51) Int. Cl.: G01N 30/88, G01N 33/28, G01N 30/74, G01N 30/06

(54) **METHOD FOR QUANTIFYING SULFUR- CONTAINING COMPOUND**
VERFAHREN ZUR QUANTIFIZIERUNG EINES SCHWEFELHALTIGEN STOFFES
PROCÉDÉ DE QUANTIFICATION D'UN COMPOSÉ CONTENANT DU SOUFRE

(30) Priority: 19.01.2024 JP 2024006833
(43) Date of publication of application: 23.07.2025
(73) Proprietor: Sumitomo Rubber Industries, Ltd., Kobe-shi, Hyogo 651-0072 (JP)
(72) Inventor: YOSHITANI, Mio, Kobe-shi, 651-0072 (JP); UNNO, Yuma, Kobe-shi, 651-0072 (JP); YAMADA, Hiroaki, Kobe-shi, 651-0072 (JP)
(74) Representative: Manitz Finsterwald Patent- und Rechtsanwaltspartnerschaft mbB

(56) References cited:
- JP-A- 2017 003 288
- JP-A- 2022 038 761
- JP-B2- 6 844 192
- DJOKIC MARKO R ET AL: "Quantitative on-line analysis of sulfur compounds in complex hydrocarbon matrices", JOURNAL OF CHROMATOGRAPHY A, ELSEVIER, AMSTERDAM, NL, vol. 1509, 2 June 2017 (2017-06-02), pages 102 - 113, XP085110339, ISSN: 0021-9673, DOI: 10.1016/J.CHROMA.2017.06.006

## Description

### TECHNICAL FIELD

The present invention relates to methods for quantifying sulfur-containing compounds.

### BACKGROUND ART

Pyrolysis gas chromatography/mass spectrometry (pyrolysis-GC/MS), for example, is a usual technique to quantify sulfur-containing compounds in compositions containing the sulfur-containing compounds. However, the sensitivity of pyrolysis-GC/MS varies daily and therefore comparison of samples measured on different measurement dates requires correction of the peak intensities with a standard material. Dibenzothiophene, which is stable against heat, is usually used as a standard material (see, Non-Patent Literature 1).

"Quantitative on-line analysis of sulfur compounds in complex hydrocarbon matrices" (DJOKIC MARKO R ET AL,JOURNAL OF CHROMATOGRAPHY A, ELSEVIER, AMSTERDAM, NL, vol. 1509, 2 June 2017, pages 102-113) discloses a method for quantifying sulfur-containing compounds, using techniques such as pyrolysis and gas chromatography, and wherein a flame detector is used.

### CITATION LIST

### NON-PATENT LITERATURE

Non-Patent Literature 1: Abstracts of the 27th National Symposium on Polymer Analysis and Characterization (2022), I-03, "Analysis of Vulcanization Mechanism of Polybutadiene by MALDI-MS and Pyrolysis GC-MS"

### SUMMARY OF INVENTION

### TECHNICAL PROBLEM

As described above, dibenzothiophene is usually used as a standard material in quantitative analysis of sulfur-containing compounds. When analyzing multiple samples, an automatic sampler is usually used for operational efficiency. Diligent studies of the present inventors revealed that since dibenzothiophene is a sublimable substance, dibenzothiophene set in an automatic sampler volatilizes over time, so that its peak area decreases. Thus, dibenzothiophene is not appropriate as a standard material in the case of using an automatic sampler.

Then, the present inventors examined the use of alkylthiophene, which is stable against heat and is not a sublimable substance, as a standard material in quantitative analysis of sulfur-containing compounds. The present inventors conducted pyrolysis-GC/MS analysis using a solution of alkylthiophene dissolved in an organic solvent. The result revealed a large variation in the peak areas of samples, indicating that the use of alkylthiophene as a standard material in quantitative analysis of sulfur-containing compounds has room for improvement.

The present invention aims to solve the above problem and provide a method for quantifying sulfur-containing compounds enabling long-term stable measurement.

### SOLUTION TO PROBLEM

The present invention is defined in the appended claims and relates to a method for quantifying sulfur-containing compounds, including use of an alkylthiophene-containing rubber composition as a standard material.

### ADVANTAGEOUS EFFECTS OF INVENTION

The method for quantifying sulfur-containing compounds of the present invention includes use of an alkylthiophene-containing rubber composition as a standard material. The method enables long-term stable measurement and can compare the amounts of a sulfur-containing compound in a composition containing the sulfur-containing compound measured at different points in time such as different measurement dates.

### BRIEF DESCRIPTION OF DRAWINGS

FIG. 1 illustrates an example of an outline of a pyrolysis gas chromatography system (Py-Gc system).
FIG. 2 illustrates a chromatogram obtained in Reference Example 1.
FIG. 3 illustrates a graph showing the result of Reference Example 3.
FIG. 4 illustrates a graph showing the result of Reference Example 4.

### DESCRIPTION OF EMBODIMENTS

The present disclosure provides a method for quantifying sulfur-containing compounds, including use of an alkylthiophene-containing rubber composition as a standard material. This method enables long-term stable measurement and can compare the amounts of a sulfur-containing compound in a composition containing the sulfur-containing compound measured at different points in time such as different measurement dates.

The reason for the advantageous effect is not exactly clear, but it is believed to be as follows. For example, in pyrolysis-GC/MS analysis using a rubber composition prepared by kneading alkylthiophene with rubber as a standard material, a variation in the pyrolysis of the standard material (the rubber composition) is small. Therefore, a variation in the peak area decreases, thereby enabling long-term stable measurement. Thus, presumably, the method can compare the amounts of a sulfur-containing compound in a composition containing the sulfur-containing compound measured at different points in time such as different measurement dates.

The rubber composition to be used as a standard material in the present disclosure at least contains alkylthiophene and a rubber component.

The alkylthiophene may be any compound in which at least one alkyl group is bound to thiophene. Examples of the alkylthiophene include a compound represented by the following formula (1): 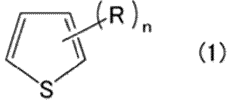 wherein R represents an optionally substituted C1-C30 alkyl group, n represents an integer of 1 to 4, and multiple Rs may be the same as or different from each other.

In the formula (1), the number of carbon atoms of R is preferably 2 or more, more preferably 5 or more, still more preferably 7 or more, further preferably 10 or more, while it is preferably 27 or less, more preferably 25 or less, still more preferably 23 or less, further preferably 20 or less, particularly preferably 16 or less.

Specific examples of R in the formula (1) include chain-like alkyl groups which may be branched, such as a methyl group, an ethyl group, a propyl group, an isopropyl group, an n-butyl group, a sec-butyl group, a t-butyl group, a pentyl group, a hexyl group, a heptyl group, an octyl group, a nonyl group, a decyl group, a undecyl group, a dodecyl group, a tridecyl group, a tetradecyl group, a pentadecyl group, a hexadecyl group, a heptadecyl group, an octadecyl group, a nonadecyl group, an eicosanyl group, a heneicosanyl group, a tetracosanyl group, a nonacosanyl group, and a triacontanyl group.

Examples of the optional substituent of the alkyl group include alkoxy groups such as a methoxy group, an ethoxy group, and a butoxy group; alkenyl groups such as a vinyl group, an allyl group, and a butenyl group; aryl groups such as a phenyl group, a naphthyl group, and a biphenyl group; alkyl groups such as a methyl group, an ethyl group, a butyl group, and a t-butyl group; an ether bond, and an ester bond.

In the formula (1), n represents an integer of 1 to 4, preferably 1, 2, or 3, more preferably 1 or 2, still more preferably 1.

In the formula (1) in which n is an integer of 1 to 3, the position of R bound to the thiophene is not limited.

To better achieve the advantageous effect of the present disclosure, the compound represented by the formula (1) is preferably, for example, a compound represented by the following formula (1-1): 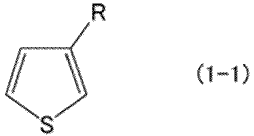
wherein R represents an optionally substituted C1-C30 alkyl group.
R in the formula (1-1) is as described for the R in the formula (1).

To particularly better achieve the advantageous effect of the present disclosure, the compound represented by the formula (1) is particularly preferably a compound represented by the following formula (1-2):

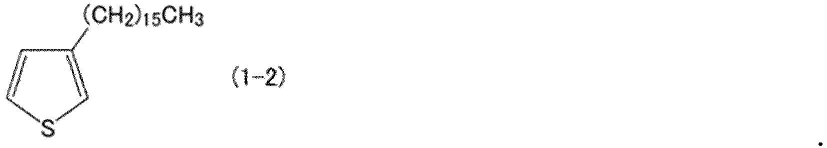

Non-limiting examples of usable rubber components include diene-based rubbers. Examples of diene-based rubbers include isoprene-based rubbers, polybutadiene rubber (BR), styrene-butadiene rubber (SBR), styreneisoprene-butadiene rubber (SIBR), ethylene-propylene-diene rubber (EPDM), chloroprene rubber (CR), and acrylonitrile-butadiene rubber (NBR). The examples also include butyl-based rubbers and fluororubber. Each of these may be used alone, or two or more of these may be used in combination.

The amount of the alkylthiophene in the rubber composition is not limited. To better achieve the advantageous effect of the present disclosure, the amount of the alkylthiophene per 100 parts by mass of the rubber component in the rubber composition is preferably 0.1 parts by mass or more, more preferably 0.5 parts by mass or more, still more preferably 1 part by mass or more, further preferably 1.5 parts by mass or more, while it is preferably 10 parts by mass or less, more preferably 7 parts by mass or less, still more preferably 5 parts by mass or less, further preferably 3 parts by mass or less.

The rubber composition may contain components other than the alkylthiophene and the rubber component within the range not impairing the advantageous effect of the present disclosure. Still, the amounts of other components are desirably as small as possible. Specifically, the amount of other components based on 100% by mass of the rubber composition is preferably 1% by mass or less, more preferably 0.5% by mass or less, still more preferably 0.1% by mass or less, further preferably 0.01% by mass or less.

The rubber composition may be produced by a known kneading method, for example, by kneading the above-described components in a rubber kneader such as an open roll mill or a Banbury mixer.

In the present disclosure, the rubber composition is subjected to, for example, pyrolysis-GC/MS analysis as a standard material. The rubber composition (standard material) is preferably finely cut and accurately weighed before the analysis. The use of such a rubber composition as a standard material can reduce the variation in the pyrolysis of the standard material, thereby reducing a variation in the peak area. Thus, presumably, the method enables long-term stable measurement and can compare the amounts of a sulfur-containing compound in a composition containing the sulfur-containing compound measured at different points in time such as different measurement dates. The weight of the cut piece of the standard material (rubber composition) is preferably 1 g or less, more preferably 100 mg or less, still more preferably 10 mg or less, further preferably 1 mg or less, further preferably 500 µg or less, further preferably 300 µg or less. The lower limit is, for example, preferably 1 µg or more, more preferably 10 µg or more.

The cut standard material (rubber composition) may be prepared by any method, for example, by a known method such as cutting or scraping the rubber composition with scissors or a razor.

The cut standard material (rubber composition) may be in any shape.

The method for quantifying sulfur-containing compounds of the present disclosure preferably includes pyrolysis gas chromatography. Such a method can quantify a sulfur-containing compound in a composition containing the sulfur-containing compound. The sulfur-containing compound can be quantified by subjecting a measurement specimen (sample) to pyrolysis gas chromatography to obtain a pyrogram and then determining the quantity from the peak area or peak height in the pyrogram. The quantification of a sulfur-containing compound in a composition containing the sulfur-containing compound by pyrolysis gas chromatography may be performed by a known method.

The term "pyrolysis gas chromatography" refers to a method involving instantly pyrolyzing a measurement specimen (sample), subjecting the pyrolysis product to gas chromatography, and separating the target component based on the difference in retention time in a column.

The pyrolizer used to pyrolyze the measurement specimen may be a device usually usable in the field. The temperature to pyrolyze the measurement specimen (pyrolysis temperature) is usually within a range of 450°C to 700°C, preferably within a range of 550°C to 650°C.

The detector to detect the component separated by the gas chromatography is not limited. For example, a mass spectrometer such as a Time-of-Flight (TOF) mass spectrometer or a flame photometric detector (FPD) for sulfur may be used. In particular, the detector is preferably a flame photometric detector for sulfur that can selectively detect sulfur.

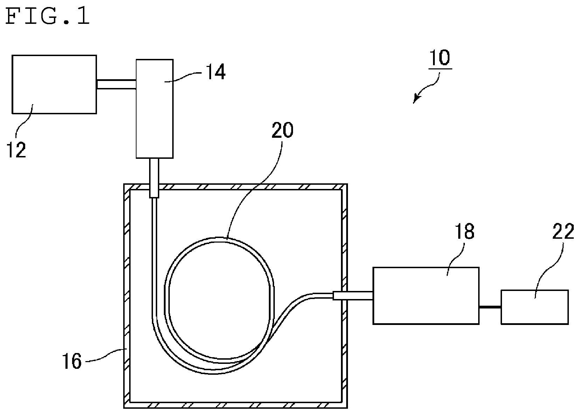

FIG. 1 illustrates an example of an outline of a pyrolysis gas chromatography system (Py-Gc system) that can be used in the present disclosure. A Py-Gc system 10 includes a carrier gas container 12, a pyrolizer 14, a gas chromatograph 16, and a detector 18. The gas chromatograph 16 includes a built-in separation column 20. The detector 18 is connected to an output device 22. Though not illustrated, the gas chromatograph 16 usually includes a means of controlling the flow rate of carrier gas and a temperature control device.

Examples of usable Py-Gc system 10 include, but not limited to, pyrolizer "EGA/PY-3030D" available from Frontier Laboratories Ltd, gas chromatograph "7890" available from Agilent, and Time-of-Flight mass spectrometer "Xevo G2-XS" available from Waters Corporation.

The measurement specimen having undergone Py-Gc analysis is put into the pyrolizer 14. The measurement specimen heated and decomposed in the pyrolizer 14 generates gas. The gas contains decomposed products of multiple components derived from organic matter in the measurement specimen.

The gas generated by the measurement specimen is introduced to the separation column 20 with carrier gas supplied from the carrier gas container 12. The separation column 20 separates multiple components in the gas. The components separated by the separation column 20 are sequentially detected by the detector 18. The detector 18 converts the amounts of the detected components to electrical signals and outputs the signals as a pyrogram from the output device 22**.** The detection time and the signal intensity are on the horizontal axis and vertical axis, respectively, of the pyrogram.

The output pyrogram shows multiple peaks. Each peak corresponds to a component in the gas generated by the measurement specimen. The area or height of the peak correlates with the amount of the component. Preferable examples of the method to calculate the amount of the component include, but not limited to, an internal standard method based on the peak area or peak height. According to the internal standard method, the standard material (rubber composition) and the measurement specimen together are subjected to pyrolysis gas chromatography. The peak area or peak height corresponding to alkylthiophene in the standard material (rubber composition) is standardized by the amount of the analyzed standard material (rubber composition). The standardized peak area or peak height corresponding to alkylthiophene is compared with the peak area or peak height corresponding to the sulfur-containing compound in the gas generated by the measurement specimen, whereby the sulfur-containing compound in the measurement specimen can be quantified.

The sulfur-containing compound to be quantified is not limited. The quantification method of the present disclosure can quantify any sulfur atom-containing compound.

As described above, the method of the present disclosure uses an alkylthiophene-containing rubber composition as a standard material. Thus, the method enables long-term stable measurement and can compare the amounts of a sulfur-containing compound in a composition containing the sulfur-containing compound measured at different points in time such as different measurement dates.

### EXAMPLES

The present disclosure is specifically described with reference to examples, but the present disclosure is not limited to the examples.

The chemicals used in examples and comparative examples are listed below.
3-Hexadecyl thiophene: a product available from Tokyo Chemical Industry Co., Ltd.
Rubber: BR150 available from UBE Corporation
Organic solvent: acetone available from Yoneyama Yakuhin Kogyo Co., Ltd.
Dibenzothiophene: a product available from Tokyo Chemical Industry Co., Ltd.

### (Preparation Example 1: Preparation of rubber composition containing 3-hexadecyl thiophene)

An amount of 0.3 g of the 3-hexadecyl thiophene and 150 g of the rubber were kneaded with a roll to prepare a rubber composition.

### (Preparation Example 2: Preparation of 3-hexadecyl thiophene solution)

An amount of 200 µg of the 3-hexadecyl thiophene was put into 1 mL of the organic solvent and stirred to prepare a 3-hexadecyl thiophene solution.

### (Preparation Example 3: Preparation of target component)

An amount of 0.3 g of N-cyclohexylbenzothiazole-2-sulfenamide (CZ available from Ouchi Shinko Chemical Industrial Co., Ltd.) and 150 g of the rubber (BR150 available from UBE Corporation) were mixed with a roll to prepare a target component.

### (Example 1)

An amount of 200 µg of the target component prepared in Preparation Example 3 and 200 µg of the rubber composition prepared in Preparation Example 1 were put into the same sample cup and subjected to Py-Gc analysis described below. Table 1 shows the results (peak intensity ratio and quantified value).

### <Py-Gc analysis>

Py-Gc analysis was performed under the conditions described below to obtain a pyrogram. Of the pyrolysis products, 3-hexadecyl thiophene, dibenzothiophene, and benzothiazole were detected from the pyrogram and the peak areas thereof were determined.

### (Conditions)

Pyrolizer: vertical micro electric furnace-type pyrolizer "EGA/PY-3030D" available from Frontier Laboratories Ltd.
Pyrolysis temperature: 550°C
Gas chromatograph: gas chromatograph "7890" available from Agilent (The measurement was performed while setting the temperatures of an interface heater and a specimen inlet (end of an inlet of specimen) to 250°C and using a temperature-increasing program that includes keeping an oven temperature at 40°C for three minutes, then increasing the temperature from 40°C to 300°C at a rate of temperature increase of 8°C/min, and maintaining the temperature at 300°C for 15 minutes. A head pressure was set to 83 kPa in a constant pressure mode, and a split ratio was set to 50:1.)
Detector: Time-of-Flight mass spectrometer "Xevo G2-XS" available from Waters Corporation (The measurement conditions include a corona current of 2.0 µA and a cone voltage of 40V.)
Detector: flame photometric detector for sulfur "5380" available from O·I·Analytical (measurement condition: detector temperature of 250°C)
Carrier gas: helium
Column: capillary column "UltraAlloy⁺-5 (MS/HT)" available from Frontier Laboratories Ltd. (5% diphenyl and 95% dimethylpolysiloxane, 30 m × 0.25 mm, I.D. × 1.0 µm film)

### (Comparative Example 1)

Py-GC analysis was performed as in Example 1, except that 200 µg of the target component prepared in Preparation Example 3 and 2 µL of the 3-hexadecyl thiophene solution prepared in Preparation Example 2 were put into the same sample cup. Table 1 shows the results (peak intensity ratio and quantified value).

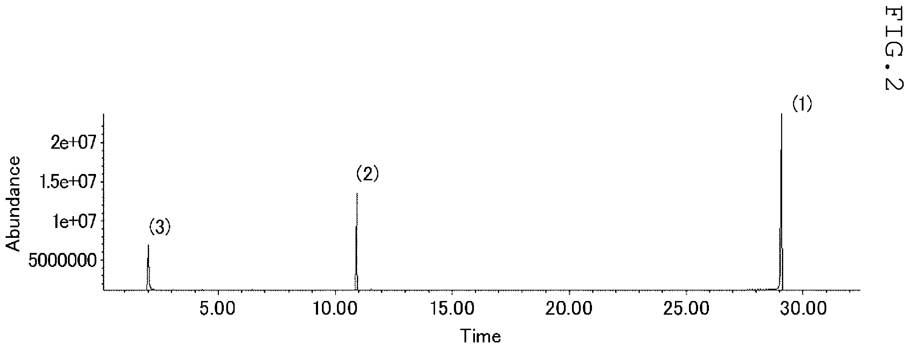

The peak intensity ratios in Table 1 were calculated with the following equation: 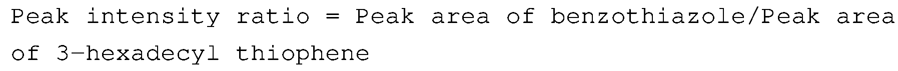 Peak intensity ratio = Peak area of benzothiazole/Peak area of 3-hexadecyl thiophene wherein the peak area of 3-hexadecyl thiophene is a total peak area of the peaks (1) to (3) in the chromatogram in FIG. 2.

Then, the quantified values were calculated with the following equation: 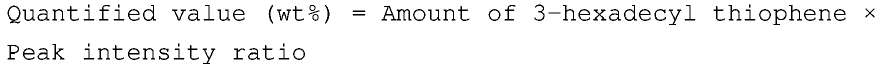 Quantified value (wt%) = Amount of 3-hexadecyl thiophene × Peak intensity ratio wherein the amount of 3-hexadecyl thiophene is the amount (wt%) of the 3-hexadecyl thiophene in the rubber composition prepared in Preparation Example 1 or the amount (wt%) of the 3-hexadecyl thiophene in the solution prepared in Preparation Example 2.

**[Table 1]**

| | Peak intensity ratio | Quantified value |
|---|---|---|
| Example 1 | 0.52 | 0.10 wt% |
| Comparative Example 1 | 0.65 | 0.13 wt% |

### (Reference Example 1)

Five sample cups each containing about 200 µg of the rubber composition prepared in Preparation Example 1 were prepared. The cups were subjected to Py-Gc analysis as in Example 1 (measurement interval between samples: 50 minutes). As a result, a chromatogram as illustrated in FIG. 2 was obtained. The variation coefficient (CV value) of the peak area (peak intensity) of the 3-hexadecyl thiophene was 3.9%.

### (Reference Example 2)

Ten sample cups each containing 2 µL of the 3-hexadecyl thiophene solution prepared in Preparation Example 2 were prepared. The cups were subjected to Py-Gc analysis as in Example 1 (measurement interval between samples: 50 minutes). The variation coefficient (CV value) of the peak area (peak intensity) of the 3-hexadecyl thiophene was 25%.

The results of Reference Example 1 and Reference Example 2 demonstrate that the variation in the peak area (peak intensity) of 3-hexadecyl thiophene is larger in the 3-hexadecyl thiophene solution prepared in Preparation Example 2 than the variation in the samples of the rubber composition containing 3-hexadecyl thiophene prepared in Preparation Example 1.

### (Reference Example 3)

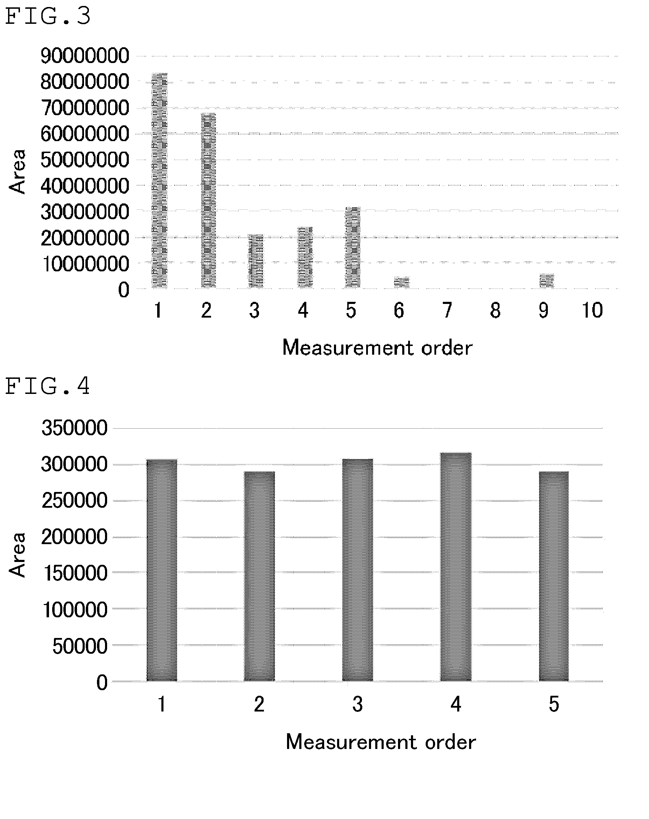

An amount of 250 µg of the dibenzothiophene was put into 1 mL of the organic solvent and stirred to prepare a dibenzothiophene solution. Ten sample cups each containing 2 µL of the dibenzothiophene solution were prepared. The cups were set in an autosampler and sequentially subjected to Py-Gc analysis as in Example 1 (measurement interval between samples: 50 minutes) to determine the peak area of dibenzothiophene. As a result, a graph illustrated in FIG. 3 was obtained. The result in FIG. 3 demonstrates that the peak area of dibenzothiophene greatly varies among the measured samples, with the tendency that the later the measurement order is, specifically, the longer the time kept in the automatic sampler is, the more the peak area decreases.

### (Reference Example 4)

Five sample cups each containing about 200 µg of the rubber composition prepared in Preparation Example 1 were prepared. The cups were set in an automatic sampler and sequentially subjected to Py-Gc analysis as in Example 1 (measurement interval between samples: 50 minutes). Of the peak areas of 3-hexadecyl thiophene, the peak area of the peak (1) in the chromatogram in FIG. 2 was determined. As a result, a graph illustrated in FIG. 4 was obtained. The result in FIG. 4 demonstrates that 3-hexadecyl thiophene has a small variation in the peak area among the samples thereof and that 3-hexadecyl thiophene does not reduce its peak area even when it is measured on a later order, specifically, after it is long kept in the automatic sampler. Comparison with the peak area of the peak (2) or (3) in the chromatogram in FIG. 2 revealed that the variation coefficient (CV value) was about 3 to 4%.

Exemplary embodiments of the present invention include the following.

Embodiment 1. A method for quantifying sulfur-containing compounds, including use of an alkylthiophene-containing rubber composition as a standard material.

Embodiment 2. The method for quantifying sulfur-containing compounds according to Embodiment 1, including pyrolysis gas chromatography.

Embodiment 3. The method for quantifying sulfur-containing compounds according to Embodiment 1 or 2, including use of a flame photometric detector for sulfur as a detector.

### REFERENCE SIGNS LIST

- 10: pyrolysis gas chromatography system
- 12: carrier gas container
- 14: pyrolizer
- 16: gas chromatograph
- 18: detector
- 20: separation column
- 22: output device

## Claims

1. A method for quantifying sulfur-containing compounds, **characterized in that** said method comprises using an alkylthiophene-containing rubber composition as a standard material.

2. The method for quantifying sulfur-containing compounds according to claim 1, comprising pyrolysis gas chromatography.

3. The method for quantifying sulfur-containing compounds according to claim 1 or 2, comprising use of a flame photometric detector for sulfur as a detector.

## Patentansprüche

1. Verfahren zur Quantifizierung schwefelhaltiger Verbindungen, **dadurch gekennzeichnet, dass** das Verfahren die Verwendung einer Alkylthiophen-haltigen Kautschukzusammensetzung als Standardmaterial umfasst.

2. Verfahren zur Quantifizierung schwefelhaltiger Verbindungen nach Anspruch 1, welches Pyrolyse-Gaschromatographie umfasst.

3. Verfahren zur Quantifizierung schwefelhaltiger Verbindungen nach Anspruch 1 oder 2, welches die Verwendung eines flammenphotometrischen Detektors für Schwefel als Detektor umfasst.

## Revendications

1. Procédé de quantification de composés contenant du soufre, **caractérisé en ce que** ledit procédé comprend l'utilisation d'une composition de caoutchouc contenant un alkylthiophène comme matériau standard.

2. Procédé de quantification de composés contenant du soufre selon la revendication 1, comprenant une chromatographie en phase gazeuse par pyrolyse.

3. Procédé de quantification de composés comprenant du soufre selon la revendication 1 ou 2, comprenant l'utilisation d'un détecteur photométrique de flamme pour le soufre comme détecteur.
